(19) Europäisches Patentamt
European Patent Office
Office européen des brevets

(11) Veröffentlichungsnummer : **0 205 031**
**B1**

# (12) EUROPÄISCHE PATENTSCHRIFT

(45) Veröffentlichungstag der Patentschrift : **17.08.88**

(51) Int. Cl.⁴ : **C 07 C101/22**, C 07 C 99/00

(21) Anmeldenummer : **86107161.1**

(22) Anmeldetag : **27.05.86**

## (54) Verfahren zur Herstellung von 4-Benzyl-aspartat.

(30) Priorität : **11.06.85 DE 3520808**

(43) Veröffentlichungstag der Anmeldung : **17.12.86 Patentblatt 86/51**

(45) Bekanntmachung des Hinweises auf die Patenterteilung : **17.08.88 Patentblatt 88/33**

(84) Benannte Vertragsstaaten : **CH DE GB LI**

(56) Entgegenhaltungen :
**DE-A- 2 608 174**
**HOUBEN-Weyl "Methoden der organischen Chemie", 4. Auflage, Band XV/1, Synthese von Peptiden Teil I, 1974 GEORG THIEME VERLAG, Stuttgart**

(73) Patentinhaber : **HOECHST AKTIENGESELLSCHAFT**
**Postfach 80 03 20**
**D-6230 Frankfurt am Main 80 (DE)**

(72) Erfinder : **Schlingmann, Merten, Dr.**
**Schneidhainer Strasse 32a**
**D-6240 Königstein/Taunus (DE)**
Erfinder : **Hoppe, Hans-Ulrich Dr.**
**Habichtsweg 4**
**D-6238 Hofheim am Taunus 3 (DE)**
Erfinder : **Dürsch, Walter, Dr.**
**In der Braubach 4**
**D-6240 Königstein/Taunus (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

L-4-Benzyl-aspartat ist von Interesse als Zwischenprodukt für eine Synthese des Süßstoffs L-α-Aspartyl-L-phenyl-alaninmethylester (« Aspartam »). 4-Benzyl -aspartat wird, wie in der US-Patentschrift 4,394, 308 beschrieben, bisher direkt aus Asparaginsäure, Benzylalkohol und Schwefelsäure nach einem Verfahren hergestellt, bei dem große Mengen an Ethanol, Diethylether und einer organischen Base, wie Pyridin oder Triethylamin, verwendet werden. Dieser Weg kann wegen großer, unvermeidbarer Lösungsmittelverluste und Umweltbeeinträchtigungen im technischen Maßstab nur unter großem apparativen Aufwand beschritten werden.

Auch verschiedene andere Verfahren, die von L-Asparaginsäure-dibenzylester ausgehen, weisen erhebliche Nachteile auf, wie z. B. die Notwendigkeit des Einsatzes von Kupfersalzen bzw. von Lithiumhydroxid, (J. Org. Chem., 40, 3287, 1975 ; Synthesis, 744, 1982 ; Deutsche Offenlegungsschrift 2 608 174), die einen technischen Einsatz sehr erschweren oder verteuern.

Es bestand daher Interesse an einem einfachen Verfahren zur Herstellung von 4-Benzyl-aspartat, das sowohl umweltschonend als auch technisch problemlos durchzuführen ist.

Es ist bekannt, daß bei der hydrogenolytischen Abspaltung von Benzylesterresten unter Bildung von Toluol freie Carboxylgruppen entstehen. Es konnte jedoch nicht erwartet werden, daß die Hydrierung von Asparaginsäure-dibenzylester und dessen Salzen überwiegend in Richtung des 4-Benzylaspartats gelenkt werden kann, zumal aus Asparaginsäuredibenzylester-tosylat unter speziellen Bedingungen auch das 1-Benzyl-aspartat erhalten wird. (Houben-Weyl, 15/1, S. 349).

Überraschenderweise wurde nun ein einfaches Verfahren zur Herstellung von 4-Benzyl-aspartat gefunden, indem, ausgehend von Asparaginsäure-dibenzylester bzw. seinen Salzen, unter ausgewählten Bedingungen selektiv nur eine Benzylgruppe, vornehmlich der 1-Benzylrest, abhydriert wird. Es entsteht ein Gemisch von Produkten, aus dem das gewünschte Produkt durch Ausfällung abgetrennt wird.

Die Erfindung betrifft somit ein Verfahren zur Herstellung der Verbindung der Formel I,

$$C_6H_5-CH_2-O-\overset{\overset{O}{\|}}{C}-CH_2-\underset{NH_2}{\underset{|}{CH}}-\overset{\overset{O}{\|}}{C}-OH \qquad (I)$$

und ihrer Salze, das dadurch gekennzeichnet ist, daß eine Verbindung der allgemeinen Formel II,

$$C_6H_5-CH_2-O-\overset{\overset{O}{\|}}{C}-CH_2-\underset{NH_2}{\underset{|}{CH}}-\overset{\overset{O}{\|}}{C}-O-CH_2-C_6H_5\cdot(HZ)_m \qquad (II)$$

in der Z ein anorganisches oder organisches Säure-Äquivalent und m 0 oder 1 bedeuten, in einem wäßrigen und/oder organischen Medium katalytisch hydriert wird.

In der folgenden Beschreibung und den Patentansprüchen werden bevorzugte Ausgestaltungen der Erfindung aufgeführt.

Asparaginsäure-dibenzylester kann in Form eines Tosylats einfach, analog Houben-Weyl 15/1, S. 348, durch azeotrope Entwässerung von Asparaginsäure und Benzylalkohol mit Benzol- bzw. p-Toluolsulfonsäure als Katalysator und Toluol als Wasserschlepper in guten Ausbeuten hergestellt werden. Aus dem derart entstandenen Asparaginsäure-dibenzylestertosylat kann der basische Diester durch alkalische Verbindungen freigesetzt und auch in andere Salze überführt werden.

Die verschiedenen Salze des Asparaginsäure-dibenzylesters bzw. die freie Base können als Ausgangsprodukte sowohl als einheitliche Substanzen wie auch in Mischung eingesetzt werden. Bevorzugt sind Asparaginsäure-dibenzylester-tosylat wegen der problemlosen direkten Zugänglichkeit, sowie die Salze mit Benzolsulfonsäure.

Als Verdünnungsmittel kommen je nach Art des verwendeten Ausgangsproduktes Wasser und/oder wassermischbare Lösungsmittel wie niedere Alkohole, Tetrahydrofuran, Dioxan, Aceton, Acetonitril, aber auch wasserunlösliche Lösungsmittel, wie die Ester Methyl-, Ethyl bzw. n-Butylacetat oder die Kohlenwasserstoffe oder Chlorkohlenwasserstoffe wie Toluol, Chlorbenzol oder Methylenchlorid in Betracht. Bevorzugt sind Wasser oder einwertige Alkohole mit 1 bis 3 Kohlenstoffatomen, gegebenenfalls im Gemisch mit Wasser, ferner Toluol, weil es durch die Reaktion auch entsteht.

Die Wahl des Verdünnungsmittels wird von einer möglichst guten Löslichkeit des verwendeten Ausgangsprodukts bestimmt. Die anorganischen Salze des Asparaginsäure-dibenzylesters, aber auch z. B. sein Acetat und sein Formiat sind in Wasser und niederen Alkohole gut löslich, während sie in hydrophoben Lösungsmitteln nur schwer zu lösen sind. Die Salze mit Benzolsulfonsäure bzw. p-Toluolsulfonsäure lösen sich in heißem Wasser wie auch in erwärmten niederen Alkoholen und den

erwärmten Gemischen niederer Alkohole mit Wasser. Der basische freie Diester wird von den meisten organischen Lösungsmitteln, nicht jedoch von Wasser, aufgenommen.

Die Ausgangsverbindung sollte schon zu Beginn möglichst vollständig gelöst sein. Ist dies nicht möglich, so kann ein Teil in Form von Lösung nachdosiert werden. Das Reaktionsgemisch sollte während der Reaktion wenigstens vorübergehend homogen werden, um eine bessere Umsetzung des Diesters sowie eine gute Ausbeute von 4-Benzyl-aspartat zu erzielen, indem die Entstehung von unerwünschter Asparaginsäure und 1-Benzyl-aspartat unterdrückt wird.

Es wird mit Wasserstoff in Mengen von 0,7 bis 1,3 Mol, bevorzugt 0,9 bis 1,1 Mol, hydriert. Als Hydrierkatalysatoren sind z. B. die im Organikum (1970) S. 314 aufgeführten Metallkatalysatoren Palladium, Palladiumchlorid, Platin, Raney-Nickel geeignet. Bevorzugt ist Palladium-Kohle mit Palladiumgehalten von 5 bis 10 %. Es sind 0,1-2,0 %, bevorzugt 0,2-1,0 %, an Palladiumkohle (mit 5 % Palladium), bezogen auf das Ausgangsprodukt, notwendig. Die Hydrierung kann in allen üblichen Hydriervorrichtungen im Laboratoriums-, Technikums- und Produktionsmaßstab problemlos durchgeführt werden.

Die Wasserstoffüberdrucke können zwischen ca. 0,01 und 50 bar, bevorzugt zwischen 0,02 und 2 bar schwanken. Die Reaktionstemperaturen können sich zwischen —30 °C und 150 °C, bevorzugt 0 °C und 80 °C, bewegen. In den meisten Fällen ist eine Temperatur von 20-60 °C besonders geeignet. Die Reaktionszeiten liegen zwischen 10 Minuten und 48 Stunden, bevorzugt zwischen 30 Minuten und 16 Stunden. Bei Anwesenheit von Wasser kann bei höheren Temperaturen teilweise hydrolytische Abspaltung von Benzylalkohol auftreten.

Die anfallenden Reaktionsprodukte sind je nach Verfahrensweise durch mehr oder weniger große Mengen an 1-Ester, Diester, Benzylalkohol und Toluol verunreinigt und müssen davon befreit werden. Die Art der Reinigung ist abhängig von der Art des Ausgangsproduktes und des Verdünnungsmittels. Wird von einem Salz des Asparaginsäure-dibenzylesters ausgegangen, so resultieren durch die Freisetzung einer Carboxylgruppe pro Molekül stark saure Salze des 4-Benzyl-aspartats. Diese sind in Wasser und/oder wassermischbaren Lösungsmitteln gut löslich. Das gewünschte 4-Benzyl-aspartat kann aus derartigen Lösungen nach dem Absaugen des Hydrierkatalysators durch Zugabe von Neutralisationsmitteln, z. B. durch Alkali- bzw. Erdalkalihydroxide, -methylate, -carbonate bzw. -bicarbonate, bevorzugt Natronlauge, als das am schwersten lösliche innere Salz bei pH-Werten von ca. 2-7, bevorzugt 3-6, selektiv ausgefällt werden. Ist nach dem Absaugen und Trocknen der Niederschläge der gewünschte Reinheitsgrad noch nicht erreicht, so können die eventuell noch vorhandenen Verunreinigungen an 1-Benzyl- bzw. Dibenzylester durch Umkristallisation aus der 12-20-fachen Menge Wasser entfernt werden.

Geringe Mengen an Verunreinigungen können auch durch Kalt- oder Heißextraktion aus dem schwerer löslichen 4-Ester mit der ca. 2-5-fachen Wassermenge beseitigt werden. pH-Werte unter 2 und vor allem über 6 oder 7 sollten besonders bei höheren Temperaturen längerzeitig vermieden werden, da sie zur hydrolytischen Abspaltung des Benzylesters führen können.

Wird vom freien basischen Diester ausgegangen, so resultiert durch die Hydrierung direkt das 4-Benzyl-aspartat, gegebenenfalls neben etwas 1-Benzyl- und Dibenzyl-asparaginsäure-ester, als ein in den meisten Lösungsmitteln schwer lösliches inneres Salz. Dieses kann z. B. zusammen mit dem ebenfalls unlöslichen Hydrierkatalysator abgesaugt werden. Im Filtrat verbleiben nur geringe Mengen an Benzylalkohol neben dem Verdünnungsmittel und dem durch die Reaktion entstandenen Toluol. Der graue Filterkuchen kann entweder aus Wasser umkristallisiert werden oder z. B. durch molare Mengen an Salzsäure als 4-Benzyl-aspartat-hydrochlorid gelöst und nach dem Absaugen des unlöslichen Hydrierkatalysators durch Einstellen eines pH-Wertes von 2-7, bevorzugt 3-6, wieder ausgefällt und abgesaugt werden.

Das noch feuchte, gereinigte 4-Benzyl-aspartat kann in üblicher Weise, z. B. im Exsikkator oder im Vakuumtrockenschrank, bei einem Vakuum von 50-400 mbar und Temperaturen von 40-100 °C, bevorzugt 50-90 °C, getrocknet werden.

Die Reinheit des resultierenden 4-Benzyl-aspartats kann durch 1H-NMR-Spektroskopie und/oder HPLC-Analysen überprüft werden.

Das erfindungsgemäße Verfahren ist umweltschonend und technisch problemlos durchführbar. Ein derart hergestelltes L-4-Benzyl-aspartat von hinreichender Reinheit kann z. B. direkt für die Herstellung von Aspartam eingesetzt werden.

Die Erfindung soll durch die folgenden Beispiele näher erläutert, jedoch nicht eingeschränkt werden. In einer Vielzahl der Beispiele wird die Ausgangsverbindung in der L-Form eingesetzt. Selbstverständlich können auch die D- bzw. D,L-Form verwendet werden. Die stereoisomeren Formen und das Racemat sind beliebig gegeneinander austauschbar.

## Beispiele

Die HPLC-Analyse wird bei allen Beispielen an « reversed phase »-Kieselgel, das mit Alkylresten mit 8 C-Atomen silyliert ist (RP8), mit einem Fließsystem aus Methanol/Wasser (1 : 1, v : v) bei einem pH-Wert von 2,8 durchgeführt.

### Beispiel 1

In einer Laboratoriumshydrierapparatur wird die Mischung aus 97,1 g (0,2 Mol) L-Asparaginsäure-dibenzylester-tosylat, 200 g Methanol, 100 g Wasser und 0,8 g Palladium-Kohle (5 %ig an Palladium) unter Stickstoff auf 45 °C erhitzt. Dabei geht das Salz in Lösung. Es wird evakuiert, das Vakuum mit Wasserstoff aufgehoben und die gleiche Prozedur wiederholt. Anschließend werden 4 600 ml Wasserstoff bei 40 °C innerhalb von 100 Minuten eingeleitet. Nach Absaugen des Hydrierkatalysators wird mittels einer Glaselektrode durch Zugabe von 25,5 g 33 %iger Natronlauge ein pH-Wert von 5,5 eingestellt. Es resultiert ein farbloser Niederschlag, der abgesaugt und zweimal mit je 50 ml Wasser gewaschen wird. Nach dem Trocknen im Vakuumtrockenschrank bei 70 °C erhält man 31,6 g Roh-L-4-Benzyl-aspartat, das durch Verreiben mit 200 ml Wasser weiter gereinigt wird. Nach dem Absaugen und Trocknen des unlöslichen Rückstandes im Vakuumtrockenschrank bei 70 °C resultieren 26,4 g (59,1 % der Theorie) reines L-4-Benzyl-aspartat gemäß H-NMR und HPLC.

Beispiel 2

Man verfährt nach Beispiel 1, setzt jedoch als Verdünnungsmittel eine Mischung aus 300 g Isopropanol und 120 g Wasser ein. Bei 45 °C werden die 4 600 ml Wasserstoff in 50 Minuten aufgenommen. Es resultieren 29,8 g Rohprodukt bzw. 24,4 g (54,7 % d.Th.) reines L-4-Benzyl-aspartat.

Beispiel 3

Man verfährt analog Beispiel 1, setzt jedoch als Verdünnungsmittel eine Mischung aus 200 g Tetrahydrofuran und 200 g Wasser ein. Es wird bei 28 °C 120 Minuten hydriert. Mit 28,4 g 33 %iger Natronlauge wird ein pH-Wert von 6 eingestellt. Nach dem Trocknen erhält man 21,2 g Rohprodukt bzw. 19,2 g (43,0 % d. Th.) reines L-4-Benzyl-aspartat.

Beispiel 4

Man verfährt gemäß Beispiel 1, setzt jedoch als Verdünnungsmittel 300 g Methanol ein. Es wird mit 4 600 ml Wasserstoff bei 40 °C in 40 Minuten hydriert. Nach dem Absaugen des Hydrierkatalysators werden bei Raumtemperatur 36,0 g einer 30 %igen Lösung von Natriummethylat in Methanol zugetropft. Der entstandene farblose Niederschlag wird abgesaugt und zweimal mit je 60 g Methanol gewaschen. Nach dem Trocknen erhält man 29,1 g (65,7 % d.Th.) reines L-4-Benzyl-aspartat.

Beispiel 5

In einer Laboratoriumshydrierapparatur wird die Mischung aus 48,6 g (0,1 Mol) L-Asparaginsäure-dibenzylestertosylat, 150 g Aceton, 100 g Wasser und 0,4 g Palladium-Kohle (5 %ig an Palladium) unter Stickstoff auf 30 °C erhitzt. Nachdem das Tosylat in Lösung gegangen ist, wird evakuiert, das Vakuum mit Wasserstoff aufgehoben und diese Maßnahme wiederholt. Nach der Aufnahme von 2 300 ml Wasserstoff, die nach 120 Minuten beendet ist, wird der Hydrierkatalysator abgesaugt und der pH-Wert des Filtrats mit 14,0 g 33 %iger Natronlauge auf 5,6 gestellt. Nach dem Absaugen und Trocknen im Vakuumtrockenschrank bei 70 °C werden 9,1 g (40,7 % d.Th.) an L-4-Benzyl-aspartat erhalten.

Beispiel 6

Man verfährt gemäß Beispiel 5, setzt jedoch als Verdünnungsmittel ein Gemisch aus 100 g Dioxan und 100 g Wasser ein. Es wird mit 2 300 ml Wasserstoff bei 45 °C in 150 Minuten hydriert. Nach dem Absaugen des Hydrierkatalysators werden 14,7 g 33 %ige Natronlauge zugefügt. Es resultieren ein pH-Wert von 5,6 und ein Niederschlag, der mit 50 ml Wasser verrieben, abgesaugt und im Vakuumtrockenschrank bei 70 °C getrocknet wird. Man erhält 10,7 g (47,9 % d.Th.) reines L-4-Benzyl-aspartat.

Beispiel 7

Man verfährt gemäß Beispiel 5, setzt jedoch als Verdünnungsmittel 300 g Wasser von 60 °C ein. Das Tosylat ist zu Beginn der Hydrierung nur teilweise gelöst, geht aber im Laufe der Aufnahme von 2 300 ml Wasserstoff, die in insgesamt 6 Stunden erfolgt, in Lösung. Das Reaktionsprodukt besteht aus drei Phasen. Die mittlere wäßrige Hauptphase wird isoliert und mit 12,0 g 33 %iger Natronlauge auf einen pH-Wert von 5,5 gestellt. Der farblose Niederschlag wird abgesaugt, mit 60 ml Wasser verrieben, wieder abgesaugt und in Vakuumtrockenschrank bei 70 °C getrocknet. Man erhält 11,0 g (49,3 % d.Th.) reines L-4-Benzyl-aspartat.

Beispiel 8

Man verfährt gemäß Beispiel 5, setzt jedoch als Verdünnungsmittel 150 g Acetonitril und 50 g Wasser ein. Es wird mit 2 300 ml Wasserstoff bei 30 °C in 80 Minuten hydriert. Nach dem Absaugen des

Katalysators wird mit 14,6 g 33 %iger Natronlauge der pH-Wert 5,5 eingestellt. Der Niederschlag wird mit 50 ml Wasser verrieben, wieder abgesaugt und getrocknet. Es resultieren 5,8 g (26 % d.Th.) reines L-4-Benzyl-aspartat.

Wegen der relativ guten Löslichkeit von L-4-Benzyl-aspartat in dem Lösungsmittelgemisch wird das Filtrat im Wasserstrahlvakuum bis auf 72 g eingeengt. Nach der Zugabe von 50 ml Wasser wird der Niederschlag abgesaugt, mit 50 ml Wasser verrieben und wieder abgesaugt. Das gleiche wird nochmals wiederholt. Der dann anfallende Filterrückstand wird getrocknet. Man erhält weitere 3,6 g (16,1 % d.Th.) reines L-4-Benzyl-aspartat.

## Beispiel 9

Man verfährt gemäß Beispiel 5, setzt jedoch als Verdünnungsmittel ein Gemisch aus 100 g Toluol und 100 g Wasser ein. Es wird mit 2 300 ml Wasserstoff bei 50 °C in 90 Minuten hydriert. Nach dem Absaugen des Katalysators wird die Toluolphase abgetrennt. Die wäßrige Unterphase wird mit 14,8 g 33 %iger Natronlauge auf den pH-Wert 5,7 gestellt. Der ausgefallene Niederschlag wird mit 50 ml Wasser verrieben und nach dem Absaugen im Vakuumtrockenschrank bei 70 °C getrocknet. Man erhält 12,6 g (56,5 % d.Th.) reines L-4-Benzyl-aspartat.

## Beispiel 10

97,1 g (0,2 Mol) L-Asparaginsäure-dibenzylester-tosylat, 250 g Toluol, 200 g Wasser und 89,7 g (0,25 Mol) 4 M Natronlauge werden in einem Scheidetrichter intensiv vermischt. Nach der Phasentrennung wird die untere wäßrige Phase nochmals mit 100 g Toluol extrahiert und der Toluolextrakt zur ersten Toluol-Oberphase gegeben. Zu den vereinigten Toluolphasen, welche den basischen Diester enthalten, Kommen 0,4 g Palladium-Kohle (5 % Pd). Anschliessend wird auf 50 °C erhitzt und der Kolbeninhalt nach dem im Beispiel 1 beschriebenen Verdrängen des Stickstoffs durch Wasserstoff innerhalb von 21 Stunden mit 4 500 ml Wasserstoff hydriert. Nach 8 und 16 Stunden werden nochmals je 0,2 g Hydrierkatalysator zugefügt. Das ausgefallene rohe L-4-Benzyl-aspartat wird zusammen mit dem Katalysator abgesaugt. Der Filterkuchen wird vollständig von Toluol befreit und in 600 ml heißem Wasser aufgenommen. Nach dem Abtrennen des unlöslichen Katalysators kristallisiert reines L-4-Benzyl-aspartat aus. Es wird abgesaugt und im Vakuumtrockenschrank bei 70 °C getrocknet. Man erhält 22,5 g (50,4 % d.Th.) reines L-4-Benzyl-aspartat.

## Beispiel 11

Man verfährt gemäß Beispiel 10, jedoch werden der Toluolphase vor dem Hydrieren noch 100 g Wasser beigemengt. Es wird mit 4 500 ml Wasserstoff bei 25 °C in 120 Minuten hydriert. Das rohe L-4-Benzyl-aspartat wird zusammen mit dem Katalysator abgesaugt und das Produkt aus 600 ml heißem Wasser von 90 °C umkristallisiert. Es resultieren nach dem Absaugen und Trocknen 20,4 g (45,7 % d.Th.) reines L-4-Benzyl-aspartat.

## Beispiel 12

Man verfährt gemäß Beispiel 10, fügt jedoch zu der Toluolphase vor der Hydrierung noch 80 ml Wasser und 13,3 g (0,05 Mol) 4 M Salzsäure hinzu. Die Hydrierung erfolgt bei 30 °C und ist nach 110 Minuten beendet. Das ausgefallene Rohprodukt wird aus 600 ml Wasser umkristallisiert. Man erhält 21,0 g (47,0 % d.Th.) reines L-4-Benzyl-aspartat.

## Beispiel 13

Man verfährt gemäß Beispiel 10, setzt jedoch der Toluolphase vor der Hydrierung noch 53,1 g (0,2 Mol) Salzsäure hinzu. Die Hydrierung erfolgt bei 28 °C und ist nach 100 Minuten beendet. Nach Zugabe von 100 ml Wasser wird der Katalysator abgesaugt und das Filtrat mit 63,1 g 4 M Natronlauge auf einen pH-Wert von 4,0 gestellt. Der ausgefallene Niederschlag wird abgesaugt und im Vakuumtrockenschrank bei 70 °C getrocknet. Es resultieren 22,3 g (49,9 % d.Th.) reines L-4-Benzyl-aspartat.

## Beispiel 14

3,5 g (10 mMol) L-Asparaginsäure-dibenzylester-hydrochlorid, 20 g Wasser, 20 ml (20 mMol) 1 M Salzsäure und 0,1 g Palladium-Kohle (5 % Palladiumgehalt) werden bei 25 °C in 160 Minuten mit 230 ml Wasserstoff hydriert. Nach Absaugen des Hydrierkatalysators werden 7,6 g 4 M Natronlauge zugegeben. Dabei fällt ein Niederschlag aus, der abgesaugt und getrocknet wird.

Man erhält 1,04 g (46,6 % d.Th.) reines L-4-Benzyl-aspartat.

## Beispiel 15

17,5 g (50 mMol) D,L-Asparaginsäure-dibenzylester-hydrochlorid, 130 g Wasser, 2,5 ml (10 mMol) 4 M Salzsäure und 0,2 g Palladium-Kohle (5 % Pd) werden bei 35 °C mit 1 160 ml Wasserstoff in 170 Minuten hydriert. Nach Absaugen des Hydrierkatalysators wird das Filtrat mit 17,2 g 4 M Natronlauge auf den pH-Wert von 5,8 gestellt. Der Niederschlag wird abgesaugt und getrocknet. Man erhält 6,5 g (58,2 % d.Th.) reines D,L-4-Benzyl-aspartat.

Beispiel 16

Man verfährt gemäß Beispiel 5, verwendet jedoch D,L-Asparaginsäure-dibenzylester-tosylat und 100 g Methanol und 100 g Wasser als Verdünnungsmittel. Es wird mit 2 300 ml Wasserstoff bei 40 °C in 110 Minuten hydriert. Der Hydrierkatalysator wird abgesaugt und das Filtrat mit 13,2 g 33 %iger Natronlauge auf einen pH-Wert von 5,6 gestellt. Der farblose Niederschlag wird abgesaugt. Nach dem Trocknen im Vakuumtrockenschrank bei 70 °C erhält man 11,0 g (49,3 % d. Th.) reines D,L-4-Benzyl-aspartat.

**Patentansprüche**

1. Verfahren zur Herstellung der Verbindung der Formel I,

$$C_6H_5-CH_2-O-\overset{O}{\overset{\|}{C}}-CH_2-\underset{NH_2}{\underset{|}{C}}H-\overset{O}{\overset{\|}{C}}-OH \qquad (I)$$

und ihrer Salze, dadurch gekennzeichnet, daß eine Verbindung der allgemeinen Formel II,

$$C_6H_5-CH_2-O-\overset{O}{\overset{\|}{C}}-CH_2-\underset{NH_2}{\underset{|}{C}}H-\overset{O}{\overset{\|}{C}}-O-CH_2-C_6H_5\,'(HZ)_m \qquad (II)$$

in der Z ein anorganisches oder organisches Säureäquivalent und m 0 oder 1 bedeuten, in einem flüssigen wäßrigen und/oder organischen Medium katalytisch hydriert wird.

2. Verfahren nach Anspruch 1, dadurch gekennzeichnet, daß eine Verbindung der allgemeinen Formel II verwendet wird, in der Z Chlorid oder der Rest der Benzol- oder p-Toluolsulfonsäure bedeutet.

3. Verfahren nach Anspruch 1 oder 2, dadurch gekennzeichnet, daß als flüssiges Medium Wasser und/oder Alkohole mit 1 bis 3 Kohlenstoffatomen eingesetzt werden.

4. Verfahren nach einem oder mehreren der Ansprüche 1-3, dadurch gekennzeichnet, daß mit 0,7 bis 1,3 Mol Wasserstoff bei einem Überdruck von 0,01 bis 50 bar und einer Reaktionstemperatur von — 30° bis 150 °C hydriert wird.

5. Verfahren nach Anspruch 4, dadurch gekennzeichnet, daß mit 0,9 bis 1,1 Mol Wasserstoff bei einem Überdruck von 0,02 bis 2 bar und einer Reaktionstemperatur von 0 bis 80 °C hydriert wird.

6. Verfahren nach Anspruch 5, dadurch gekennzeichnet, daß bei einer Reaktionstemperatur von 20 bis 60 °C hydriert wird.

7. Verfahren nach einem oder mehreren der Ansprüche 1-6, dadurch gekennzeichnet, daß die Verbindung der Formel I durch Fällen bei pH-Werten von 2 bis 7 isoliert wird.

8. Verfahren nach Anspruch 7, dadurch gekennzeichnet, daß die Verbindung der Formel I durch Fällen bei pH-Werten von 3 bis 6 isoliert wird.

**Claims**

1. A process for the preparation of the compound of the formula I

$$C_6H_5-CH_2-O-\overset{O}{\overset{\|}{C}}-CH_2-\underset{NH_2}{\underset{|}{C}}H-\overset{O}{\overset{\|}{C}}-OH \qquad (I)$$

and of its salts, which comprises catalytic hydrogenation of a compound of the formula II

$$C_6H_5-CH_2-O-\overset{O}{\overset{\|}{C}}-CH_2-\underset{NH_2}{\underset{|}{C}H}-\overset{O}{\overset{\|}{C}}-O-CH_2-C_6H_5\cdot(HZ)_m \qquad (II)$$

in which Z is an inorganic or organic acid equivalent, and m is 0 or 1, in a liquid aqueous and/or organic medium.

2. The process as claimed in claim 1, wherein use is made of a compound of the formula II in which Z denotes chloride or the radical of benzenesulfonic or p-toluenesulfonic acid.

3. The process as claimed in claim 1 or 2, wherein the liquid medium used is water and/or alcohols having 1 to 3 carbon atoms.

4. The process as claimed in one or more of claims 1-3, wherein hydrogenation is carried out with 0.7 to 1.3 mol of hydrogen under a gage pressure of 0.01 to 50 bar and at a reaction temperature of —30° to 150 °C.

5. The process as claimed in claim 4, wherein hydrogenation is carried out with 0.9 to 1.1 mol of hydrogen under a gage pressure of 0.02 to 2 bar and at a reaction temperature of 0 to 80 °C.

6. The process as claimed in claim 5, wherein hydrogenation is carried out at a reaction temperature of 20 to 60 °C.

7. The process as claimed in one or more of claims 1-6, wherein the compound of the formula I is isolated by precipitation at pH values of from 2 to 7.

8. The process as claimed in claim 7, wherein the compound of the formula I is isolated by precipitation at pH values of from 3 to 6.

**Revendications**

1. Procédé pour préparer le composé de formule I

$$C_6H_5-CH_2-O-\overset{O}{\overset{\|}{C}}-CH_2-\underset{NH_2}{\underset{|}{C}H}-\overset{O}{\overset{\|}{C}}-OH \qquad (I)$$

et ses sels, procédé caractérisé en ce qu'on hydrogène catalytiquement un composé répondant à la formule générale II

$$C_6H_5-CH_2-O-\overset{O}{\overset{\|}{C}}-CH_2-\underset{NH_2}{\underset{|}{C}H}-\overset{O}{\overset{\|}{C}}-O-CH_2-C_6H_5\cdot(HZ)_m \qquad (II)$$

dans laquelle Z représente un équivalent d'acide minéral ou organique, et m est égal à 0 ou à 1, dans un milieu liquide aqueux et/ou organique.

2. Procédé selon la revendication 1 caractérisé en ce qu'on utilise un composé de formule générale II dans lequel Z représente le chlore ou le radical de l'acide benzène-sulfonique ou p-toluène-sulfonique.

3. Procédé selon l'une des revendications 1 et 2, caractérisé en ce qu'on utilise, comme milieu liquide, l'eau et/ou des alcools contenant de 1 à 3 atomes de carbone.

4. Procédé selon l'une quelconque des revendications 1 à 3, caractérisé en ce qu'on hydrogène avec de 0,7 à 1,3 mol d'hydrogène, sous une surpression de 0,01 à 50 bar et à une température réactionnelle de —30 à 150 °C.

5. Procédé selon la revendication 4 caractérisé en ce qu'on hydrogène avec de 0,9 à 1,1 mol d'hydrogène, sous une surpression de 0,02 à 2 bars et à une température réactionnelle de 0 à 80 °C.

6. Procédé selon la revendication 5 caractérisé en ce qu'on hydrogène à une température réactionnelle de 20 à 60 °C.

7. Procédé selon l'une quelconque des revendications 1 à 6, caractérisé en ce qu'on isole le composé de formule I par précipitation à des pH de 2 à 7.

8. Procédé selon la revendication 7 caractérisé en ce qu'on isole le composé de formule I par précipitation à des pH de 3 à 6.